# EUROPEAN PATENT SPECIFICATION

(11) **EP 1 950 562 B1**
(45) Date of publication and mention of the grant of the patent: **13.07.2016**
(21) Application number: 08075192.8
(22) Date of filing: 02.04.2003
(51) Int. Cl.: A61B 5/00, A61B 5/15, G01N 33/487, A61B 5/145, A61B 5/157

(54) **Integrated sample testing meter**
Integrierter Probentestmesser
Compteur de test d'échantillon intégré

(30) Priority: 02.04.2002 GB 0207610
(43) Date of publication of application: 30.07.2008
(62) Divisional of application: 03712439.3
(73) Proprietor: Lifescan Scotland Limited, Inverness-Shire IV2 3ED (GB)
(72) Inventor: Griffith, Alun Wyn, Iverness IV2 2XQ (GB); May, Keith, Ashford Kent, TN26 3PU (GB); Diston, Andrew Stephen, Cambridge CB4 6AJ (GB); Drought, Nicholas Andrew, Milton Cambridge, CB4 6DS (GB); Wilson, Donna Joy, New York NY 10128 (US)
(74) Representative: Tunstall, Christopher Stephen

(56) References cited:
- EP-A- 1 112 717
- WO-A-02/08753
- WO-A-02/18940
- WO-A-94/10558
- WO-A-02/078533
- CA-A1- 2 245 943
- US-A- 5 759 010
- US-A- 6 036 924

## Description

### Field of the Invention

The present invention relates to an integrated sample testing meter for use in sampling and analyzing analytes, particularly glucose, in fluids such as blood or interstitial fluid.

### Background of the Invention

Glucose monitoring is a fact of everyday life for diabetic individuals. The accuracy of such monitoring can literally mean the difference between life and death. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

Many of the glucose meter designs currently available make use of a disposable test strip which, in combination with the meter, electrochemically or photometrically measures the amount of glucose in the blood sample. To use these meters, the user first punctures a finger or other body part using a puncture means, such as a lancet, to produce a small sample of blood or interstitial fluid. The sample is then transferred to a disposable test strip. The inconvenience of taking several measurements a day, as well as the pain inflicted by currently available puncture means, often discourage disciplined and frequent testing.

While the fingertip is generally used for sampling blood, due to the rich capillary bed of the skin of the fingertip, the fingertip is also particularly sensitive to pain, due to a rich supply of pain receptors in the finger tip as well. When a puncture is too deep, too close to a recent puncture or not deep enough and requires an additional puncture, the pain increases significantly. Pain may also be increased if the puncture means penetrates slowly or is withdrawn slowly. Furthermore, the user may be forced to make a larger puncture than is necessary to form a sufficient amount of blood, due to losses during the transfer between the puncture site and the test strip.

The process of measuring blood glucose levels requires several steps and several different accessories, including a puncturing device, a puncture means, a supply of test strips and a glucose meter. Each accessory has a different function. The user must have a flat surface available to unpack and lay down the accessories within easy reach. This, by itself, poses a challenge for those who need to take measurements while participating in outdoor activities. Flat surfaces are often not available and this can discourage a person from taking a measurement. This can be disadvantageous because blood glucose levels may change significantly during an outdoor activity.

Even if a user can find a flat surface, the user has to carry out the following steps. The user: charges the puncturing device with a fresh puncture means; opens a vial of strips; removes a strip; inserts the strip into the meter; re-closes the vial; checks for the correct calibration code on the meter; picks up the puncturing device; lances the skin of the finger or other body part; lays down the puncturing device; squeezes or massages the finger to yield an adequate blood sample; transfers the sample to the test strip for analysis; waits four the meter to analyze the sample; removes the strip from the test meter; discards the strip; and finally re-packs all of the accessories. As set forth above, the standard procedure for taking a glucose measurement requires the use of multiple, separate components and the execution of a number of steps requiring manual user intervention.

Generally, the user is required to transfer a small volume of sample to a sample-receiving area on the test strip. Generally, test strips are quite small and the sample-receiving area is therefore even smaller. This transfer step is a difficult task for many users. Moreover, there has recently been a trend towards the use off test strips requiring ever smaller amounts off sample. (This allows the use of smaller punctures and therefore less painful puncturing.) However, the use of smaller samples increases the difficulty in transferring the sample to the sample-receiving area on the test strip. This is especially difficult for users with poor eyesight, a common complication for diabetics.

The pain, inconvenience, cost, slowness, complexity and discreteness of taking a blood glucose measurement are barriers to the frequent monitoring of glucose levels. Patients often do not comply with doctor recommendations to frequently test glucose levels due to the numerous obstacles involved.

It is an aim of the present invention to provide, at least in part, a solution to the above problems.

WO 02/18940 discusses a test device for testing of analyte concentration in a fluid comprising: a housing having an opening and containing a stack of sensors; a transport member rotatably mounted in the opening of the housing, having an axis of rotation which spans the opening; a spring which urges the stack against the transport member; and sealing means for making a moisture tight seal between the transport member and the sensors when the transport member is in a specified rotational position. An outer surface of the transport member has a recessed region which is adapted to receive a single sensor from the stack. Rotation of the transport member with a sensor in the recessed region transports the sensor to a location where it can be connected to a meter and receive a drop of fluid to be tested.

The present invention provides a cartridge for holding and dispersing test strips according to claim 1 and a method of dispersing test strips from the housing of a cartridge according to claim 11.

### Summary of the Invention

in one embodiment, which is not claimed, an integrated sample-testing meter comprises a single modular housing carrying:
a puncture means;
a drive train for driving the puncture means between an extended position and a retracted position;
a test strip cartridge containing a plurality of test strips, each strip having a sample-receiving area;
a sensor for analyzing a fluid sample received on a test strip; and
a test strip dispensing system for moving test strips individually from the cartridge to a sample-receiving position in which the test strip is connected to the sensor,
the meter being arranged such that, in use, when it is located in a first position on the skin of a user and is activated, the puncture means is moved to its extended position and retracted to form a puncture in the user's skin and a test strip is moved from the cartridge to the sample-receiving position.

In use, after the puncture has been made, the user moves the meter to a second position where the sample-receiving area of the test strip is located in the drop and receives a sample from the fluid drop. The sensor then analyzes the sample.

The meter may include electrical or electronic circuitry for controlling its operation. Such circuitry may be hard-wired or may comprise a microcomputer or similar device. Such circuitry will in particular include all the components of the sensor and will be arranged to carry out the analysis of the sample.

Preferably, the circuitry also includes a visual display unit from which the user can read out the result of any particular test. The display may also be adapted to provide a display of the data, as explained in more detail below.

Preferably, the circuitry includes means, such as a touch sensitive display, control buttons or a microphone and voice activated software, for intering data into the meter.

Preferably, the meter includes a pressure device arranged to facilitate the formation of a drop of fluid around the puncture.

The pressure device may comprise a pump adapted to apply a negative pressure to a volume in the meter having an aperture for location on the skin of the user. Advantageously, the part of the meter forming the aperture through which the puncture means extends is made of a non-slip material so that the meter can be more securely located on the user's skin during the puncturing operation.

Preferably, however, the pressure device comprises a pressure ring arranged to be located, in use, on the user's skin and to apply pressure at the edges of the ring to increase the amount of fluid available at the centre of the ring. Advantageously, the pressure ring is made of a non-slip material so that the meter can be more securely located on the user's skin during the puncturing operation.

The pressure ring may be shaped to conform to the shape of the area of the skin to which it is to be applied. For instance, if the meter is intended for use on the forearm, the pressure ring will be generally planar. However, if the meter is intended for use on a finger, the pressure ring will be curved.

Preferably, the pressure ring has a multi-contoured surface to increase the pressure gradient from the outside to the inside of the ring.

Advantageously, the pressure ring is part of a cap which covers the puncture means in its retracted position. Preferably, the cap includes a means, such as a sidewall, which co-operates with the drive train to ensure that the puncture means travels along approximately the same path on each activation of the drive train.

The cap may be an integral part of the housing. Preferably, however, the cap is detachably mounted on the housing. This may be achieved by use of screw-thread or bayonet type fixings, by use of a snap fit connection or by a hinged connection.

If desired, the meter can include at least two interchangeable caps, for instance a cap for forearm use and a cap for finger use.

The puncture means may be any of the types of puncture means at present in use in the art. The term "puncture means" includes lancets and finger-sticking devices of the type known in the art. Preferably, the puncture means is removably attached to the drive train so that the puncture means can be disposed of after one or several uses.

Preferably, the drive train is spring driven. Alternatively, the drive train is driven electromagnetically. The drive train is arranged such that, on actuation, the puncture means moves to the extended position and is retracted.

Preferably, the drive train includes an adjustment screw which allows the user to set the extended position of the puncture means. This enables the user to calibrate the operation of the meter such that his or her skin is punctured sufficiently to allow a large enough drop of fluid to form without causing too much pain.

Advantageously, the operation of the adjustment screw is arranged such that the distance of travel of the puncture means remains constant, however much the extended position of the puncture means is changed. This ensures that the amount of pain experienced by the user does not increase disproportionately to the depth of puncture.

Where the meter includes a cap, it is preferable, as noted above, that the cap provides a means for guiding the drive train so that the puncture means punctures the skin at approximately the same place on each actuation of the drive train.

Preferably, the test strip cartridge comprises a cartridge housing defining a cavity configured to receive a stack of test strips, a partially detachable cartridge cap and a means for moving the stack of test strips towards the cartridge cap.

The test strips used for some measurements are air- or moisture sensitive. It is therefore preferred that the cartridge includes a seal for sealing the cartridge cap to the cartridge housing when the cartridge cap is fully engaged with the cartridge housing. The seal may be on the cartridge cap or on the cartridge housing.

In one embodiment, which is not claimed, upon activation of the meter, a cartridge cap is partially detached from the cartridge housing to allow the first test strip in the stack to be moved by the test strip dispensing system to the sample-receiving position. Once the measurement has been taken, the cartridge cap is preferably manually replaced on the cartridge housing to close the cartridge and seal its contents from atmospheric effects.

Preferably, the cartridge has on it data relating to the calibration code for the strips in the cartridge. The data may be present as visually readable indicia. In this case, the meter must include means, as mentioned above, to allow the user to enter the calibration code into the meter.

Preferably, however, the data on the cartridge are present in machine-readable format, for instance as a bar code or a resistance bridge circuit or stored in an electronic memory module.

If the data are present as a bar code, the meter will include a bar code reader. This may be a scanning reader or a stationary reader. A scanning reader will be more complicated but can be used when the cartridge is fitted in the meter. A stationary reader is less complicated but can only be used as the cartridge is inserted into or taken out of the meter.

If the data are present in an electronic memory module, this may comprise a read-only memory (ROM), or a rewritable memory, such as an EPROM or EEPROM.

Preferably, the data also include a unique number identifying the specific cartridge, the number of strips originally present in the cartridge, the expiry date for the cartridge, different calibration factors for different sources of fluid (neonatal, arterial or venous blood, for instance), an acceptable performance range and any other relevant information, preferably in machine-readable format, to assist in operation of the meter.

Where the memory module on the cartridge is rewritable, the meter may be arranged to write back into the memory module information such as the number of strips used, the date the cartridge was first used, the length of time the cartridge has remained open and the date, time and result of each test that was carried out with a strip from the cartridge.

Preferably, the test strip dispensing system includes a slider adapted to engage with only one of the test strips in the cartridge and move it to the sample-receiving position.

Advantageously, the meter includes a feeding channel which receives the strip from the cartridge and guides it to the sample-receiving position.

Preferably, the feeding channel includes a step arranged such that, when the strip has been moved past the step, the strip drops, or is forced, into the step, thereby preventing the strip from moving back towards the cartridge.

Preferably, the strip is forced into the step by springs located on the meter. Advantageously, the springs are also electrically conductive and are arranged to make electrical contact with electrodes or a conductive bar on the strip (see below).

Alternatively, the strips may be provided with cut-outs, for instance of triangular shape, which mate with spring-biased abutments which fit into the cut-outs to hold the strip in its sample-receiving position.

Advantageously, the meter includes an ejection means for ejecting a used test strip from the meter once a test has been completed. Preferably, where the cartridge includes a cartridge cap, the ejection means is operated as the cartridge cap is closed.

Preferably, the meter includes interlock means operable to prevent the test strip dispensing system moving a further test strip into the sample-receiving position while a first test strip is still in position. This is an advantageous feature as it allows the user to carry out a number of puncturing operations with the same strip in position, since, in some cases, it takes a number of puncturing operations, if necessary with adjustment of the penetration depth, to produce a drop of sufficient volume.

Preferably, the interlock means operates in conjunction with the cartridge cap. While the cartridge cap is partially detached from the cartridge housing, the interlock means blocks the normal path for the test strip dispensing system, such as the slider, and causes it to enter the cartridge cap rather than the regular path into the cartridge.

Alternatively the interlock means may comprise a separate knob for cocking the puncture means independently of dispensing a strip from the cartridge.

Preferably, the meter includes a means for verifying that a strip is in the sample-receiving position. This may comprise a reflectance meter. Generally, test strips are more or less reflective than the surfaces of the feeding channel. Therefore, a change in reflectance will indicate that a test strip is in position.

Preferably, however, the verifying means comprises an electrical system. At its simplest, each strip may have on it a conductive bar arranged to short out two electrodes on the meter. This arrangement is useful for strips arranged to carry out photometric measurements.

Strips which are arranged to carry out electrochemical measurements already include electrode systems. Thus, the verifying means may include electrical contacts on the meter which contact the electrodes on the strip. Advantageously, as noted above, the electrical contacts on the meter are preferably spring-loaded and are positioned to force the strip into the step in the feeding channel.

Advantageously, the verifying means is also used to activate fully the circuitry in the meter. The meter may normally be in a low power mode, where the only active circuitry is that used to control the verifying means. Once the verifying means has indicated that a strip is present, the meter can then automatically switch to high power mode where all its relevant circuits are functioning.

Preferably, the verifying means is also arranged to start a timer in the circuitry of the meter. The timer is stopped by the ejection of a used strip from the meter, preferably by closure of the cartridge cap. This allows the circuitry to determine the length of time the cartridge has been open to the atmosphere. Advantageously the circuitry is arranged to sum the total time that the cartridge has been open and to produce a warning signal, such as an audible tone or a visible signal, if the total exceeds a pre-set maximum.

Preferably, the circuitry in the meter also counts the number of strips dispensed from each cartridge. Advantageously, the circuitry is designed to provide a warning signal, such as an audible tone or a visible signal, when the number of strips remaining in the cartridge is low.

Where the cartridge has on it data relating to an acceptable performance range, preferably the circuitry is arranged so that, if a control test gives a result outside the performance range, the meter is disabled while that cartridge is in the meter. This arrangement ensures that, if the strips in a particular cartridge have deteriorated, they cannot be used.

As noted above, the cartridge preferably includes a rewritable memory module and the circuitry in the meter is arranged to write back to the cartridge memory module useful information, such as the number of strips remaining in the cartridge and the length of time the cartridge has been open to the atmosphere, the rewriting function is particularly useful where a user is likely to be away from his normal environment for a length of time which would require the use of more strips than are present in a cartridge. In such cases, a user is likely to remove the old cartridge and insert a new, full cartridge. Once the new cartridge has been used up, the user may insert the old cartridge, even if it is out of date. As long as the meter can read the data on the old cartridge, it will be able to decide whether use of the old cartridge should be allowed.

Moreover, the provision of a rewritable memory module enables other possible uses. For instance, data on time and date of use and result of measurement may be written into the cartridge's memory module. The used cartridge could then be returned to the user's health care provider who could then study the data to determine whether the user is complying with his treatment and monitoring regime. Alternatively, the used cartridges could be returned to the manufacturer to enable a general study of use to be carried out. These data, illustrating the effective use of strips, may provide a tool for health care insurers to verify the actual usage of the strips they reimbursed.

Preferably, the meter is activated manually by use of a single movement, for instance of a multi-functional assembly carried by the housing. The assembly may include a lever pivoted to the housing. Preferably, however, the assembly includes a slidable knob which slides to cock the drive train and move a strip into the sample-receiving position.

The movement of the assembly may also activate all the meter's circuitry.

The drive train may be fired either by further movement of the lever or, preferably, by actuation of a trigger.

It can thus be seen that the use of the integrated sample testing meter can be very simple. If desired, the user can replace an existing puncture means with a new one. The meter can then be cocked by use of the assembly. This also moves a strip into the sample-receiving position. Movement of the lever or receipt of a strip in the sample receiving position also activates all the meter's circuitry. Then, the user places the appropriate part of the meter, such as the aperture or the cap, on his or her skin and activates the trigger.

If the first activation of the trigger does not cause the production of a sufficiently large drop of fluid, the meter can be cocked, positioned and fired again, if necessary a number of times, without the need to insert a new strip.

Once sufficient fluid has accumulated around the puncture, the user moves the meter to the second position in which the strip is placed in the drop and its sample-receiving area takes up a sample of the fluid.

Thus, the use of the meter avoids most of the steps presently required and in particular avoids those steps where manual dexterity and good eyesight are advantageous.

Preferably, the meter is adapted to produce and analyze a sample of blood or interstitial fluid, in particular to analyze a blood sample for glucose levels. Strips adapted to carry out such measurements are well known in the art. These may be electrochemical or photometric strips.

Advantageously, the strips are adapted to carry out electrochemical analyses and the circuitry in the meter is arranged to contact the electrodes in such strips.

In one embodiment which is not claimed, there is provided an integrated blood glucose testing meter. The integrated meter allows for a simple, one-step glucose monitoring process, and significantly reduces the obstacles involved in frequent glucose monitoring. The integrated meter provides for the automated dispensing and positioning of a test strip and puncturing of a user in a repeatable manner. After user-initiated transfer of a blood sample to the test strip, automated analysis of the blood sample takes place.

In one embodiment, which is not claimed, there is provided an integrated method of sampling and testing a blood glucose level or other analyte in a bodily fluid. The integrated method comprises loading a test strip cartridge into an integrated testing meter, activating an assembly on the testing meter to cock a puncture means drive train and move a test strip into a sample-receiving position, pressing the integrated testing meter on the skin of a user and pressing a trigger of the testing meter to drive a puncture means into the skin in order to form a drop of blood or other fluid on the skin surface. The user moves the meter such that the test strip can absorb a required amount of blood or other fluid for an automated analysis of the sample by the integrated testing meter.

According to the present invention, there is provided a cartridge for holding and dispensing test strips comprising:
a housing having an aperture for containing test strips;
a lid slidably attached to the housing and adapted to engage a test strip from the housing;
a seal around the aperture,
wherein in a first position the lid closes the aperture and is engaged with the seal and in a second position the lid is disengaged from the seal and engaged with a test strip in the housing such that movement of the lid from the second position to the first position dispenses the test strip from the housing.

Thus, the unsealing of the lid from the housing, the dispensing of a strip from the housing and the re-sealing of the housing can be reliably achieved in a two-stage movement of the lid. There is no requirement for a separate lid and strip dispenser. Actuation of the lid and hence the sealing-dispensing-resealing process can be achieved in a single dimension of movement.

Preferably, the lid has a sliding surface juxtaposed to an engaging surface of the housing at an edge of the aperture.

Advantageously, the sliding surface has a step for engaging the test strip.

The step allows an uppermost test strip from a stack of test strips in the housing to be obtained and forced out from the housing.

In one embodiment of the present invention, at least one part of the sliding surface is sloped with respect to the engaging surface such that sliding of the lid from the first position to the second position disengages the lid from the seal. The sliding surface acts as a ramp against the engaging surface of the housing so that the lid is forced away from the seal as it slides open. Biasing means on the meter in which the cartridge is disposed bias the lid against the housing to maintain a seal between the lid and the housing in the closed position and to keep the sliding surface forced against the engaging surface as the lid is slid to its open position.

In another embodiment of the present invention, the cartridge further comprises a datum surface attached to the housing against which the lid slides and at least part of which is sloped with respect to the engaging surface of the aperture edge. The datum surface also acts as a ramp against the sliding surface of the lid.

Preferably, there is strip biasing means in the housing to bias test strips contained in the housing against the sliding surface. For example, the strip biasing means may be a helical spring or a constant-force spring.

Preferably, there is lid biasing means to bias the lid against the seal in the first position. The lid biasing means may be a leaf-spring. In one embodiment, the lid biasing means is integral with the cartridge. Alternatively, the lid biasing means may be fixed to the meter.

Preferably, the cartridge contains desiccant material in the housing. This way, moisture that enters the housing can be absorbed which is important to maintain the test strips in a good condition. Such material may be formed into a liner in the housing.

In one embodiment of the present invention, the cartridge includes identification means for holding data about the test strips in the housing.

Preferably, the identification means is a readable and writeable electronic memory chip.

The memory chip can be accessed by the meter in which the cartridge is placed or any other device suitably adapted for the same purpose.

In yet another aspect of the present invention, there is provided a method of dispensing test strips from the housing of a cartridge comprising the steps of:
sliding a lid from a first position, in which the lid closes an aperture in the housing and is engaged with a seal around the aperture, to a second position, in which the lid is disengaged from the seal and is engaged with a test strip in the housing; and
sliding the lid from the second position to the first position such that the engaged test strip is forced out of the housing by the lid.

### Brief Description of the Drawings

These and other features and advantages of the present invention will be more fully understood by reference to the following detailed description in conjunction with the attached drawings in which like reference numerals refer to like elements through the different views.
Figure 1 is a perspective view of an integrated blood and testing meter according to the present invention with a strip in the sample-receiving position.
Figure 2 is a perspective view of the meter of Figure 1 with the lancet cover in its open position.
Figure 3 is a perspective view of the meter of Figure 1 with the subhousing in its open position.
Figure 4 is a schematic view of part of the interior of a feeding channel with a strip in place.
Figure 5 illustrates a test strip design suitable for use in the present invention.
Figure 6 is a schematic representation of the electronics which can be incorporated in an integrated meter in accordance with the present invention.
Figure 7 shows a cross-section of an arrangement of a cartridge which can be used with an integrated meter in accordance with the present invention.
Figure 8 shows a cross-section of another arrangement of a cartridge which can be used with an integrated meter in accordance with the present invention.

### Detailed Description of the Invention

The present invention provides an integrated meter for sampling and analyzing a sample of bodily fluid, such as blood, including a disposable test strip cartridge having a stack of test strips disposed therein. The present invention facilitates the monitoring of, for instance, blood glucose levels by integrating into a single meter the steps involved in sampling and analyzing blood into a simple process employing a single meter.

The present invention will be described below relative to an illustrative embodiment. Those skilled in the art will appreciate that the present invention may be implemented in a number of different applications and embodiments and is not specifically limited in its application to the particular embodiment depicted herein.

The present invention will be discussed below in connection with sampling blood, although those of ordinary skill will recognize that other types of fluid can also be used.

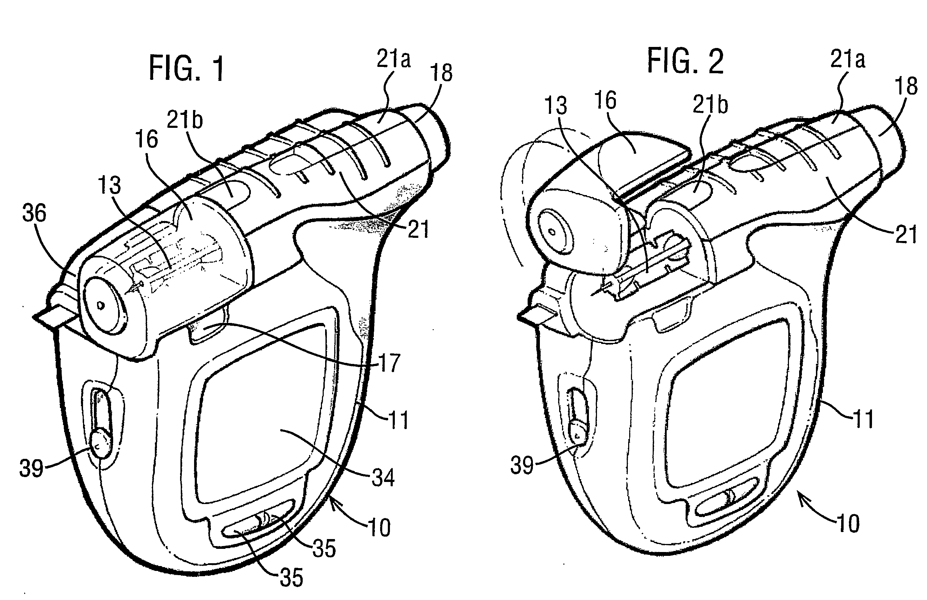

Figures 1 to 3 illustrate an integrated blood glucose sampling and testing meter 10 according to an illustrative embodiment of the present invention. This meter is designed to carry out electrochemical analysis of a blood sample. However, if desired, the same mechanical parts could be used in connection with photometric analyses. The sampling and testing meter comprises a modular housing 11 encompassing an integrated system for expressing and subsequently analyzing a sample. The meter 10 includes an assembly for puncturing the skin of a user to express a drop of blood on the surface of the skin. The assembly includes a lancet 13 as the puncture means and a drive train 14 for driving the lancet into and out of the skin.

A transparent cap 16 is attached to the housing 11 by a hinge at the proximal end of the device 10. The housing 11 includes a recess 17 for enabling the cap 16 to be moved to the open position shown in Figure 2. In this position, the lancet can be removed and replaced. The cap 16 also includes an aperture to allow passage of the lancet 13 through the cap 16 and into the skin of the user. The cap 16 can have a multi-contoured surface in order to promote, enhance or facilitate the expression of blood by pressing the device onto the skin. The assembly further includes a depth adjustment knob 18 situated at the distal end of the drive train opposite the lancet. Rotation of the depth adjustment knob decreases or increases the puncture depth of the lancet. The depth adjustment knob regulates or adjusts the puncture depth in accordance with known techniques.

A test strip cartridge 19 is loaded into the meter 10 and includes a stacked supply of test strips disposed within a cavity or hollow interior of the cartridge housing. The test strip cartridge is adapted to dispense individual test strips to a feeding channel 20. The outlet of the feeding channel leads to the exterior of the meter 10.

The housing may include an internal wall that defines the inner side of the cap 16. Alternatively, the housing wall can have a frusto-conical or funnel shape, or any other suitable shape, for precisely controlling the movement of the lancet.

The cap is precisely dimensioned such that the lancet 13 slidably passes through the cap. In this arrangement, the lancet 13 is precisely positioned at about the same location each time it is deployed. Correspondingly, each test strip is precisely positioned at about the same location each time one is moved from the cartridge to the sample-receiving position.

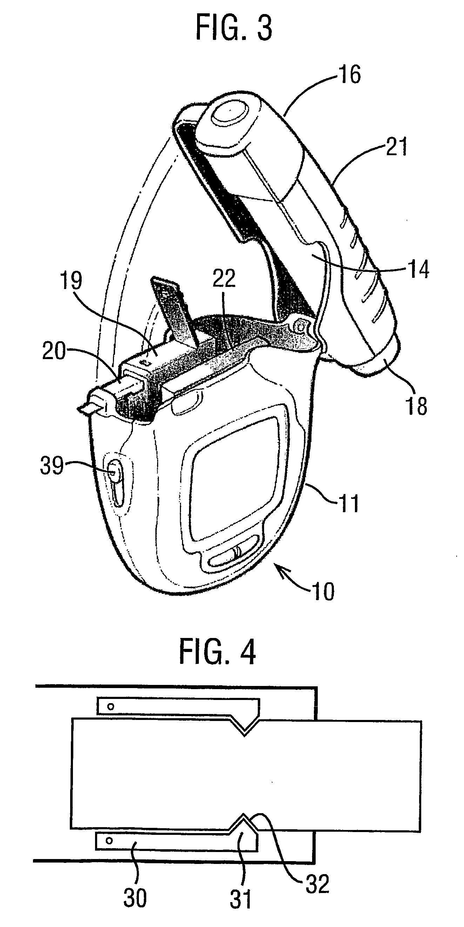

The test strip cartridge 19 comprises a replaceable and disposable portion of the sampling and testing meter. When the supply of test strips is depleted or expired, the user may open the meter 10, as shown in Figure 3, remove the used test strip cartridge 19 and insert a new test strip cartridge containing a fresh supply of test strips. The details of the test strip cartridge 19 are described in depth below.

A strip dispensing system operates in co-operation with the test strip cartridge 19 to dispense test strips one-by-one through the feeding channel 20 and into the sample-receiving position to effect the sampling and analysis of a blood sample. According to the illustrated embodiment, when a user slides knob 21 of the meter 10 away from the cap 16, the strip dispensing system pushes the foremost test strip in the stack out of the test strip cartridge and into the feeding channel. According to a preferred embodiment, knob 21 performs an additional function of simultaneously cocking the lancet assembly to prepare the lancet assembly for puncturing the skin of a user when the user presses the cap 16 to the skin. The workings of the strip dispensing system and knob 21 are described in further detail below.

To enable electrochemical analysis of the sample, the meter further includes electrical contacts situated in the feeding channel 20 and configured to contact electrodes formed on the test strip. The electrical contacts connect to electronics 22 located within the modular housing 11 of the sampling and testing meter. The electronics are arranged such that, once the contacts contact the electrodes in the strip, the meter switches from "low" power made to "high" power mode.

The test strip generates electrochemical signals that are passed by the electrical contacts to the housing electronics. The electronics process the signal and calculate the glucose level or other electrochemically detectable analyte of the blood or interstitial fluid that is sampled by the testing device. The electronics transmit instructions for an appropriate display or output regarding the analysis.

As shown in Figure 4, the feeding channel 20 has in it a pair of arms which are biased to move towards each other. Each arm has at its free end a triangular abutment 31. The strips have in them triangular cut-outs 33. When a strip is fed into the feeding channel, the abutments 31 on the arms 30 snap into the cut-outs 33 to hold the strip in the sample receiving position.

Alternatively, the feeding channel may include a step located adjacent the electrical contacts. The electrical contacts are spring biased so that, once a test strip is in the sample-receiving position, the electrical contacts bear on the test strip and locate it securely in the step.

In either manner, the strip is prevented from moving backward away from the sample-receiving position.

The integrated sampling and testing meter 10 includes a visual LCD display 34 for displaying information related to the analysis of the sample. According to the illustrative embodiment, the information in the display includes a measured blood glucose level in a blood sample, as well as the time and date of the measurement The display may also provide information regarding the number of test strips remaining in the test strip cartridge, the operating temperature, the expiration date of the test strip cartridge, instructions to the user and the like. According to one practice of the invention, test results are stored in memory in the meter and the display 34 allows a user to view prior test results.

The meter also has on its outside buttons 35 which can be used by the user to enter data into the meter's electronics. This may be achieved by using the buttons to navigate through one or more menus displayed on the display 34.

To measure blood glucose levels with the integrated meter 10, a user first slides the knob 21 away from the cap 16 to simultaneously cock the lancet assembly and automatically open the test strip cartridge and to advance a test strip from the cartridge through the feeding channel 20 to the sample-receiving position shown in Figure 1. The user then presses the cap 16 against a body part, such as a finger or forearm. This releases the lancet assembly, which fires the lancet 13 into the skin a predetermined depth and at a precise location. The lancet assembly immediately retracts the lancet from the skin. Alternatively, there maybe an independent trigger 21b to fire the lancet 13.

The cap 16 includes a pressure ring (not shown) so that, as the meter is pressed onto the skin before, during or after the puncturing has taken place, a drop of blood of the required size forms on the user's skin. The user then moves the meter to bring the sample-receiving area of the strip in the sample-receiving position into contact with the drop. As the blood contacts the sample-receiving area of the test strip, capillary force absorbs blood into the strip for analysis. The user holds the meter firmly against the skin until a sufficient amount of blood is absorbed into the test strip, generally for about 3 to 10 seconds. According to one practice, the meter 10 produces an audible or visible signal to the user indicating that a sufficient blood sample has been collected and that analysis has begun. The user then removes the meter from the skin and the electrochemical analysis of the sample continues until the result is displayed.

The disposable strip cartridge 19 includes a number of components designed to facilitate automatic, one-by-one dispensing of the test strips. The test strip cartridge includes a vial housing, a cartridge housing including a stack of test strips, a cartridge cap and a push-up or biasing mechanism. The stack of test strips comprises about fifty test strips in vertical alignment. However, the test strip cartridge of the present invention is not limited to a stack of fifty test strips and may include any number of stacked test strips.

The push-up mechanism biases the test strip stack towards the cartridge cap such that, when a foremost test strip is removed from the stack, the remaining test strips in the stack advance by one. After the foremost strip is removed from the stack, the next strip in the stack moves up and is ready to be dispensed for a subsequent analysis. The push-up mechanism includes a loader pressing against the last strip in the stack and a biasing element, such as a tensator. The tensator comprises a constant force clock spring that applies a constant pressure to the stack.

The push-up mechanism further includes a tensator retainer to secure a portion of the tensator to the cartridge housing. The vial housing further includes notches to releasably lock the cartridge in the modular housing of the meter. When loading the cartridge 19 into the meter, the vial housing clicks unambiguously in place to ensure a precise fit.

The cartridge cap includes a hermetic sealing element to protect the test strips from humidity, which can damage the test strips and compromise test results. Alternatively, the seal can be included in the vial housing where it meets the cap.

According to one practice, the cartridge material itself can have desiccant properties, or desiccants can be disposed in the interior space of the vial. Any humidity that may migrate into the test strip vial is by these materials absorbed and neutralized.

As can be seen from Figure 3, preferably the cap, the drive train, the sliding knob and a strip ejection lever (see below) are in a unitary subhousing which is pivotally attached to the remainder of the housing to allow for the insertion of a cartridge and the removal of used cartridges. The subhousing may be released by operation of release knob 39.

Preferably, the cartridge includes on it a re-writable memory module such as an EPROM or EEPROM chip. In this case, the electronics in the meter will include means for interfacing with the memory module so that the meter can read from and write to the memory module.

The memory module will contain a calibration code for the cartridge and will preferably contain a unique code for the cartridge and its expiry date. It may also contain compensation factors for analyses of different fluids (such as venous, arterial or neonatal blood or interstatial fluid), the number of strips in the cartridge and other relevant information. The electronics in the meter will be set up to use any data stored in the memory module, in particular the calibration code.

The electronics will also be set up to write to the memory module such information as the number of strips used, the length of time the cartridge cap has remained open, the date of first opening the cap, the date and times of each test carried out and the result of each test.

The cartridge may alternatively include such data in other formats, such as in visible characters, as a bar code or as a resistor bridge circuit.

The cartridge cap is releasably locked into place on the cartridge by a cap retainer. To allow for the strip dispensing mechanism in the meter to forward individual strips to the feeding channel 20, the cartridge cap includes a "pop-up" feature. The cartridge cap is flexibly attached to the vial housing by means of side supports, hinges, springs or another suitable mechanism. Pushing the cap retainer releases the lock on the cartridge cap and allows the cap to pop up a predetermined amount, thereby allowing the foremost test strip in the stack to be fed to the sample-receiving position.

The strip dispensing system cooperates with the pop-up cartridge cap described above to push the foremost test strip of a test strip stack into the feeding channel 20 in order to position the test strip in the sample-receiving position. As discussed, the strip dispensing system comprises the sliding knob 21.

Those skilled in the art will recognize that any suitable mechanism may be utilized for forwarding a test strip into a feeding channel and ensuring that the test strip is entirely dispensed. Once in the feeding channel, the test strip is positioned to receive a blood sample for analysis. After the analysis is complete, the user replaces the cartridge cap to re-seal the cartridge, for instance by operation of the ejection lever 36.

The slidable knob 21 further includes means for arming the drive train of the meter 10. According to one practice, the knob further operates to switch on the electronics of the meter 10 to prepare the meter for analysis of a prospective blood sample. According to an alternate embodiment, the electronics include a strip detector for detecting the presence of a test strip in the feeding channel. Thus, when the strip detector detects a strip in proximity to the puncturing site, the electronics switch on.

According to one aspect, the strip dispensing system is designed to ensure that only one test strip is loaded at a time. The strip dispensing system includes interlock means in co-operation with the slider. The strip dispensing system allows only one test strip to be forwarded at a time. After the slidable knob is released and the slider is brought back into its initial position, the interlock means automatically engages into a position to deflect subsequent attempts to load an additional test strip into the feeding channel. The release of the cartridge cap caused by operation of the knob allows the deviator to rotate once the slider is moved back to its idle position. After forwarding one test strip, the slider route is deviated inside the cartridge cap, rather than through the test strip cartridge and into the feeding channel of the meter 10.

When the user presses the cartridge cap closed, the interlock means disengages and resets the strip dispensing system to dispense a new strip. If a test strip is already loaded into the feeding channel 20, additional operation of the slidable knob 21 only serves to cock the lancet assembly and does not load another test strip into the channel. In this manner, the strip dispensing system allows several cocking and puncturing attempts using the same test strip. This feature is particularly useful if the lancet is accidentally discharged or if the puncturing action does not generate a sufficient amount of blood. In this case, the lancet assembly can be re-cocked without wasting a test strip. The interlock means can include an additional knob 21 a for cocking the puncture means without dispensing a strip from the cartridge 19.

The test strip cartridge 19 and the strip dispensing system co-operate with the lancet assembly illustrated in Figure 1 to efficiently and less painfully obtain and analyze a blood sample from a user. As discussed above, operation of the slidable knob 21 simultaneously cocks the lancet assembly and forwards a test strip from the cartridge into the feeding channel 20. The drive train for the lancet assembly may comprise a drive tube, a lancet holder slidably mounted in the drive tube for holding the lancet 13, a first spring for urging the lancet holder forward, a second spring for retracting the lancet 13 after the lancet punctures the skin and a depth adjuster knob 17. The lancet assembly further includes the cap 16 having an aperture for guiding the lancet 13 through the aperture to the skin of a user and for shielding the lancet when not in use.

When the slidable knob 21 is operated, the drive tube retracts to arm the lancet assembly, while simultaneously the test strip is fed through the feeding channel 20 and into the sample-receiving position shown in Figure 1. The user presses the cap 16 against a body part, such as a finger or arm, to allow the lancet assembly to drive the lancet tip into the skin. The lancet assembly subsequently retracts the lancet tip from the skin.

The pressure ring, if present, squeezes the skin to maximize the quantity of blood formed from a puncture. Once the drop of blood is large enough, the user moves the meter so that the sample-receiving area of the strip contacts the blood drop which will be wicked into the strip. The test strip automatically directs the blood sample to an analysis portion and the analysis of the blood sample begins automatically.

After the analysis is complete, the user may open the cap 16 and remove the lancet 13 from the lancet holder. The user may then discard the lancet 13, if desired. Those skilled in the art will recognize that alternate lancet assemblies may be utilized in accordance with the teachings of the present invention. For example, the present invention is not limited to the dual-spring drive train of the illustrative embodiment of the invention.

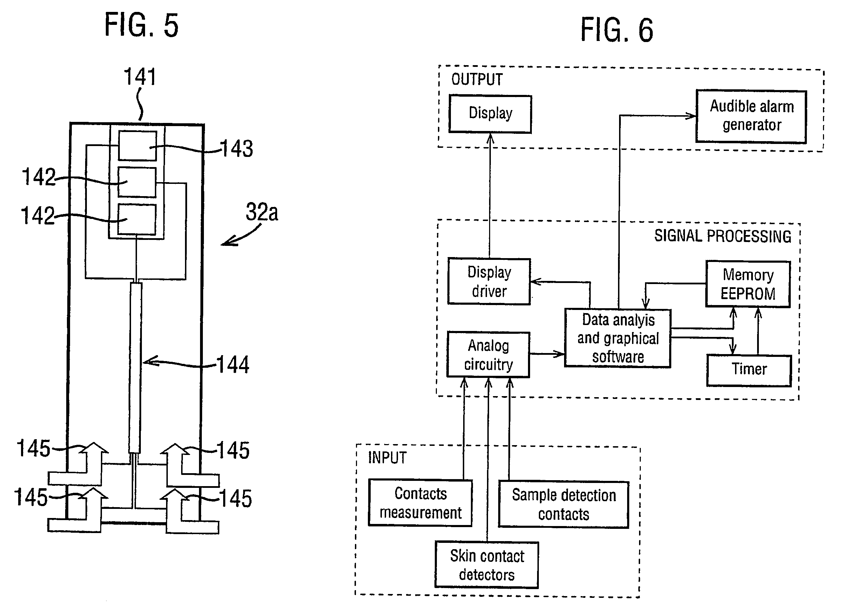

Figure 5 illustrates a test strip design suitable for use in the present invention. The test strip may utilize OneTouch Ultra (available from LifeScan, Inc. of Milpitas, CA, USA) technology, membrane strip technology or other test strip designs known in the art for electrochemical or photometric analysis of a fluid. According to one embodiment, the test strip includes, as its sample-receiving area, a channel entrance 141 for directing a blood sample to an analysis portion of the strip. The test strip essentially comprises an electrochemical cell, including one or more working electrodes 142 which convert a chemical change, produced by a reaction of glucose or other analyte in the blood sample, to a current. The test strip further includes a reference electrode 143 as a standard to measure the potential of the working electrodes. Leads 144 connect the electrodes to contact bars 145 configured to connect with the electrical contacts of the integrated testing meter. The test strip thus generates a signal indicative of the level of glucose or other analyte in the blood and transmits this signal to the electronics of the device for processing. Those skilled in the art will recognize that a variety of test strip designs and configurations are available in accordance with the teachings of the present invention.

Figure 6 shows a schematic representation of the electronics incorporated in the integrated meter of the present invention. The electronics receive a signal from the electrical contacts, process the signal and transmit instructions for an appropriate display to the display of the device. As shown, input signals related to the electrochemical analysis of the sample are provided from the test strip to a signal processing system. The signals are transmitted via analog circuitry to a processor, which performs data analysis. The processor provides a signal to a display driver connected to an output display. The processor may also provide a signal to an alarm generator. The display and the alarm generator together constitute the output portion of the device. The data analysis processor also communicates with a memory module, such as an EEPROM, in which information, including calibration information and previous test results, may be stored.

According to one practice of the invention, the electronics further include a detector for sensing a strip in the feeding channel. The detector can be two contacts which are shorted by a conductive layer on a strip when the strip is in the sample-receiving position. The electronics may be designed to produce an audible beep or visible signal to indicate to the user that a sufficient sample has been obtained and that analysis is complete. The electronics may also read, store and/or display information regarding the date and time of testing, the condition of the strips, the number of strips remaining in the stack, a calibration code for the strips, the expiration date of the test strip cartridge, the battery power of the meter, and so on. As noted above, test strip specific information can be read directly from the cartridge, for instance by use of bar codes, resistance bridges or memory modules, preferably rewritable memory modules.

As discussed, according to one embodiment, the electronics are switched on when a user slides the knob of the integrated testing meter, or when a test strip detector detects a test strip loaded in the sample-receiving position. Preferably, each time the electronics are switched on, the data on the cartridge are read to ensure that the correct calibration code and other data are used to control the meter. This ensures that a correct test result can be obtained even if the cartridge has been changed.

According to another embodiment of the invention, the electronics are switched off when the user replaces the test strip cartridge cap and ejects the used test strip from the meter. This provides an extra safety feature as it ensures that the cartridge remains closed for as long as possible. This minimises the exposure of the contents of the cartridge to the atmosphere. Preferably, the electronics in the meter are arranged to record the length of time between a strip reaching the sample-receiving position and its being ejected from the meter. This is a measure of the time the cap is open. If the total time the cap is open exceeds a predetermined value, the electronics may be arranged to provide an audible or visible warning signal. The electronics may also be arranged to provide such a signal, or to switch off the meter, if any single strip has remained in the sample-receiving position for longer than a predetermined time.

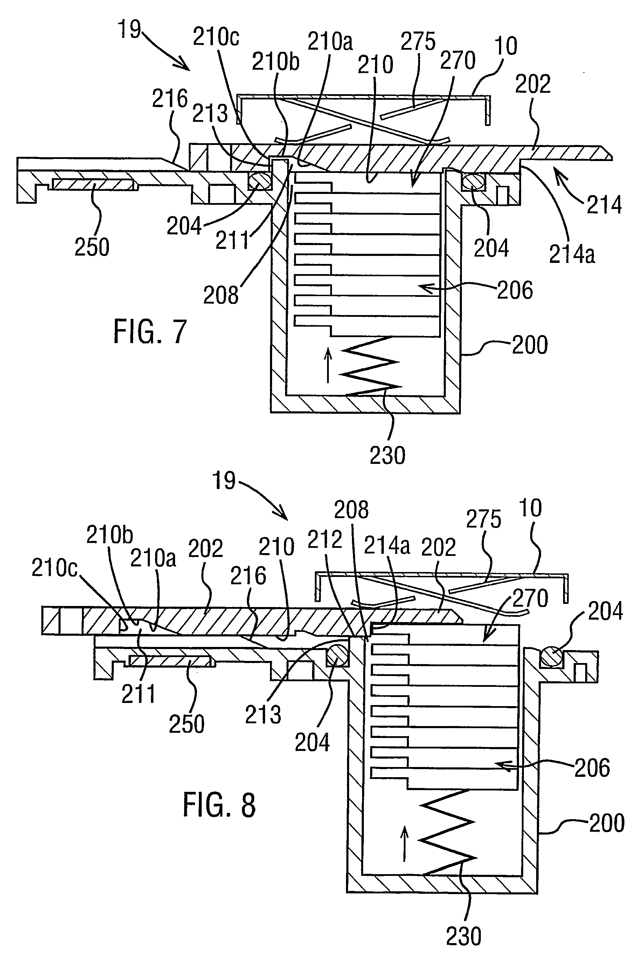

Figure 7 shows a cross-section of one embodiment of the cartridge 19 for holding and dispensing test strips 206 according to the present invention. The cartridge 19 has a housing 200 for storing a plurality of test strips 206 stacked vertically along their planar surfaces. The test strips 206 are dispensed one at a time through an aperture 208. The cartridge 19 has a lid 202 which is biased against a surface 212 of the housing at the edge of the aperture by biasing means 275 fixed to the meter 10. The lid is shown in a closed position in Figure 7. A seal 204 surrounds the aperture 208 and contacts a sliding surface 210 of the lid 202 such that the test strips 206 inside the housing 200 are hermetically sealed from the environment.

At least one section of a sliding surface 210 of the lid 202 is sloped with respect to a housing surface 212 at the edge of the aperture 208 and forms a slot 211 in the lid 202. In the closed position shown in Figure 7, the base 210b of the slot 211 is juxtaposed to the surface 212 of the housing. The lid 202 is opened, either directly or by a mechanical connection to the lancing device or other actuators of the meter 10 in which it is disposed, by sliding it over a datum surface 216 on the housing 200. As the lid is opened, the sloped section 210a of the slot 211 slides over the surface 212 of the housing 200 which raises the lid off the seal 204 in a cam-like action so that the cartridge 19 becomes unsealed. Similarly, the sliding surface 210 of the slot 211 slides over a sloped section of the datum surface 216 to raise the lid 202 away from the seal 204. Biasing means 275 biases the sliding surface 210 against the datum surface 216.

Figure 8 shows a cross-section of the cartridge of Figure 7 with the lid 202 in an open position. There is a step 214 in the sliding surface 210 at one end of the lid 202 which is adapted to receive the uppermost strip 270 from the stack of test strips 206 when the lid 202 is in its open position as shown. Strip biasing means 230 forces the stack of strips in a direction towards the lid 202. The step face 214a is juxtaposed to one end of the uppermost strip 270. As the lid 202 is slid towards its closed position, the uppermost strip 270 is forced out of the housing 200 by sliding it across the top of the stack of strips 206. The sloped section 210a of the slot 211 engages with the surface 212 at the edge of the housing 202 such that the lid 202 is lowered onto the seal 204 by the biasing action of the biasing means 275 A slot face 210c engages with a second surface 213 of the housing to prevent the lid 202 being slid beyond its closed and sealed position.

This way, the cartridge 19 of the present invention achieves the unsealing of the lid 202 from the housing 200, the disposal of a test trip and the re-sealing of the lid 202 to the housing 200 in a two-stage movement of the lid 202.

In addition, there is an electronic memory chip 250 fixed to an external surface of the housing 200. Data can be written to and read from the electronic chip. The chip stores data about the number and type of test strips 206 in the housing 200.
A more detailed description of another embodiment of the cartridge and a feeder mechanism for use therewith is found in our copending International Patent Application No. PCT/GB02/01599 (Attorney's ref: P030115WO), which claims priority from USSN 60/280321 filed on 29th March, 2001.

The integrated meter of the present invention and its components provide significant improvements to the detection and monitoring of glucose levels in the blood. The present invention considerably reduces the pain and inconvenience associated with glucose monitoring. The invention further improves the efficiency and accuracy of testing by providing an automated transfer and analysis of the sample. The invention provides an integrated testing meter with user-friendly, uncomplicated operation. The integrated testing meter is compact, ergonomically sound, discrete and adjustable to different users and body parts while simultaneously providing fast and accurate results.

The present invention achieves a reduction in the pain associated with testing in a number of ways. Shallower punctures of the skin can be used to achieve a sufficient blood sample, reducing painful deep punctures in sensitive body parts. The present invention does not require large sample volumes for analysis. The pressure device, if used, for instance formed by the pressure ring on the cap, provides a high yield from a small puncture. The integrated sampling and testing feature further ensures full usage of the obtained sample and limits "leftovers" on the skin. In current systems, complex and inaccurate sample transfer from a sampling point to a sample-receiving area on a test strip requires surplus sample due to poor utilization of an obtained sample drop. The present invention decreases this inefficiency of transferring samples and provides optimal utilization of the obtained sample by easy direction of the sample to a precise location on the test strip. Optimal utilization of the sample drop reduces the number of attempts needed to provide enough sample for efficient analysis, thus reducing the number of punctures required. The superficial punctures reduce agitation of nerve endings in the skin and reduce pain in sensitive body areas. The variable depth of penetration and the ability to test on a number of different body parts in addition to the finger reduces the concentration and repetition of micro-traumata in a small area, which avoids the problems of tinting, itching, dried and callous skin areas caused by such micro-traumata.

The integrated meter of the present invention is able fully to exploit the technological improvements in strip design which allow the use of much smaller samples. Presently available strips require only 1 to 3 µl of sample. The small volume of blood or other bodily fluid expressed from the user is sufficient to accurately determine or monitor the presence or absence of an analyte, such as glucose.

The present invention further provides easy and uncomplicated operation. The use of the meter significantly reduces the time and difficulty involved in sampling and testing blood. The integrated meter essentially provides three devices, a puncture means, a supply of test strips and a meter, within a singular compact housing. Further, the system is designed such that one-handed operation is possible, eliminating the need for a work space or a flat surface. The meter is not subject to human error and inefficiency. Furthermore, the integration of a disposable test strip cartridge makes the loading of a test strip simple, accurate and easy. In current glucose monitoring systems a user requires two hands to load a strip into a glucose meter. However, with the meter of the present invention, the test strip dispensing system automatically loads a test strip in position to receive a blood sample. The present invention also reduces waste by efficiently utilizing available resources. The present invention further protects against compromised test results due to contamination or an improperly calibrated glucose meter.

In conclusion, the integrated meter of the present invention significantly reduces the obstacles associated with frequent glucose monitoring. The present invention promotes frequent monitoring for diabetic individuals by providing a simple, efficient, fast and accurate integrated meter.

## Claims

1. A cartridge (19) for holding and dispensing test strips (206) comprising:
a housing (200) for containing test strips, the housing having an aperture (208);
a lid (202) slidably attached to the housing and adapted to engage a test strip from the housing;
wherein the lid has a sliding surface (210) juxtaposed to an engaging surface (212) at an edge of the aperture;
a seal (204) around the aperture, wherein in a first position the lid closes the aperture and is engaged with the seal and in a second position the lid is disengaged from the seal and is engaged with a test strip in the housing such that movement of the lid from the second position to the first position dispenses the test strip from the housing; and
wherein the cartridge further comprises lid biasing means to bias the lid against the seal in the first position;
**characterised in that**:
at least one section (210a) of the sliding surface is sloped with respect to the engaging surface of the aperture edge such that sliding of the lid from the
first position to the second position disengages the lid from the seal.

2. The cartridge of claim 1, wherein the sliding surface has a step (214) for engaging the test strip.

3. The cartridge of any one of claims 1 to 2 further comprising a datum surface (216) attached to the housing against which the lid slides and at least part of which is sloped with respect to the engaging surface of the aperture edge.

4. The cartridge of any one of claims 1 to 3 further comprising strip biasing means (230) in the housing to bias a stack of test strips contained in the housing against the sliding surface.

5. The cartridge of any one of claims 1 to 4 further comprising desiccant material.

6. The cartridge of any one of claims 1 to 5, further comprising identification means for holding data about the test strips in the housing.

7. The cartridge of claim 6 wherein the identification means is a readable and writeable electronic memory chip (250).

8. A method of dispensing test strips (206) from the housing (212) of a cartridge (19) comprising the steps of:
sliding a lid (202) from a first position, in which the lid closes an aperture (208) in the housing and
is engaged with a seal (204) around the aperture, to a second position, in which the lid is disengaged from the seal and is engaged with a test strip (206) in the housing;
wherein the lid has a sliding surface (210) juxtaposed to an engaging surface (212) at an edge of the aperture; and
wherein the method further comprises sliding the lid from the second position to the first position such that the test strip is forced out of the housing by the lid; and
wherein the cartridge further comprises lid biasing means to bias the lid against the seal in the first position
**characterised in that**:
at least one section (210a) of the sliding surface is sloped with respect to the engaging surface of the aperture edge such that sliding of the lid from the first position to the second position disengages the lid from the seal.

## Patentansprüche

1. Kassette (19) zum Aufnehmen und Ausgeben von Teststreifen (206), umfassend:
ein Gehäuse (200), das Teststreifen enthält, wobei das Gehäuse eine Öffnung (208) umfasst;
einen Deckel (202), der verschiebbar an dem Gehäuse befestigt ist und geeignet ist, einen Teststreifen aus dem Gehäuse zu erfassen;
wobei der Deckel eine verschiebbare Oberfläche (210) aufweist, die neben einer erfassenden Oberfläche (212) an einem Rand der Öffnung angeordnet ist;
eine Dichtung (204) um die Öffnung, wobei der Deckel in einer ersten Stellung die Öffnung verschließt und mit der Dichtung verbunden ist und wobei der Deckel in einer zweiten Stellung von der Dichtung getrennt ist und mit einem Teststreifen in dem Gehäuse so verbunden ist, dass eine Bewegung des Deckels aus der zweiten Stellung in die erste Stellung den Teststreifen aus dem Gehäuse ausgibt; und
wobei die Kassette außerdem ein Deckelspannungselement umfasst, um den Deckel in der ersten Stellung gegen die Dichtung zu drücken;
**dadurch gekennzeichnet, dass**:
mindestens ein Abschnitt (210a) der verschiebbaren Oberfläche des Öffnungsrands in Bezug auf die Erfassungsoberfläche so geneigt ist, dass das Verschieben des Deckels aus der ersten Stellung in die zweite Stellung den Deckel von der Dichtung trennt.

2. Kassette nach Anspruch 1, wobei die verschiebbare Oberfläche eine Stufe (214) für das Erfassen des Teststreifens aufweist.

3. Kassette nach einem der Ansprüche 1 oder 2, die außerdem eine Datumsoberfläche (216) umfasst, die an dem Gehäuse befestigt ist, gegen die der Deckel verschoben wird und von der mindestens ein Teil in Bezug auf die Erfassungsoberfläche des Öffnungsrands geneigt ist.

4. Kassette nach einem der Ansprüche 1 bis 3, die außerdem ein Streifenspannungselement (230) in dem Gehäuse umfasst, um einen Stapel von in dem Gehäuse enthaltenen Teststreifen gegen die verschiebbare Oberfläche zu drücken.

5. Kassette nach einem der Ansprüche 1 bis 4, die außerdem ein Trocknungsmaterial umfasst.

6. Kassette nach einem der Ansprüche 1 bis 5, die außerdem ein Identifizierungselement umfasst, das Daten über die Teststreifen in dem Gehäuse enthält.

7. Kassette nach Anspruch 6, wobei das Identifizierungselement ein lesbarer und beschreibbarer elektronischer Speicherchip (250) ist.

8. Verfahren zum Ausgeben von Teststreifen (206) aus dem Gehäuse (212) einer Kassette (19), das die folgenden Schritte umfasst:
Verschieben eines Deckels (202) aus einer ersten Stellung, in welcher der Deckel eine Öffnung (208) in dem Gehäuse verschließt und mit einer Dichtung (204) um die Öffnung verbunden ist, in eine zweite Stellung, in welcher der Deckel von der Dichtung getrennt ist und
mit einem Teststreifen (206) in dem Gehäuse verbunden ist;
wobei der Deckel eine verschiebbare Oberfläche (210) aufweist, die neben einer erfassenden Oberfläche (212) an einem Rand der Öffnung angeordnet ist; und
wobei das Verfahren außerdem ein Verschieben des Deckels aus der zweiten Stellung in die erste Stellung umfasst, sodass der Teststreifen durch den Deckel aus dem Gehäuse gezwungen wird; und
wobei die Kassette außerdem ein Deckelspannungselement umfasst, um den Deckel in der ersten Stellung gegen die Dichtung zu drücken;
**dadurch gekennzeichnet, dass**:
mindestens ein Abschnitt (210a) der verschiebbaren Oberfläche in Bezug auf die Erfassungsoberfläche so geneigt ist, dass das Verschieben des Deckels aus der ersten Stellung in die zweite Stellung den Deckel von der Dichtung trennt.

## Revendications

1. Cartouche (19) pour contenir et distribuer des bandes de test (206), comprenant:
un logement (200) pour contenir des bandes de test, le logement comportant une ouverture (208);
un couvercle (202) attaché de façon coulissante au logement et apte à engager une bande de test en provenance du logement;
dans laquelle le couvercle présente une surface de coulissement (210) juxtaposée à une surface d'engagement (212) au niveau d'un bord de l'ouverture;
un joint (204) autour de l'ouverture, dans laquelle, dans une première position, le couvercle ferme l'ouverture et est engagé avec le joint et, dans une seconde position, le couvercle est désengagé du joint et est engagé avec une bande de test dans le logement de telle sorte qu'un déplacement du couvercle depuis la seconde position jusqu'à la première position distribue la bande de test à partir du logement; et
dans laquelle la cartouche comprend en outre un moyen de poussée de couvercle pour pousser le couvercle contre le joint dans la première position;
**caractérisée en ce que**:
au moins une section (210a) de la surface de coulissement est inclinée par rapport à la surface d'engagement du bord d'ouverture de telle sorte que le coulissement du couvercle depuis la première position jusqu'à la seconde position désengage le couvercle du joint.

2. Cartouche selon la revendication 1, dans laquelle la surface de coulissement comporte un gradin (214) pour engager la bande de test.

3. Cartouche selon l'une quelconque des revendications 1 à 2, comprenant en outre une surface de référence (216) attachée au logement contre laquelle le couvercle coulisse et dont au moins une partie est inclinée par rapport à la surface d'engagement du bord d'ouverture.

4. Cartouche selon l'une quelconque des revendications 1 à 3, comprenant en outre un moyen de poussée de bande (230) dans le logement pour pousser une pile de bandes de test contenue dans le logement contre la surface de coulissement.

5. Cartouche selon l'une quelconque des revendications 1 à 4, comprenant en outre un matériau absorbeur d'humidité.

6. Cartouche selon l'une quelconque des revendications 1 à 5, comprenant en outre un moyen d'identification pour contenir des données relatives aux bandes de test dans le logement.

7. Cartouche selon la revendication 6, dans laquelle le moyen d'identification est une puce mémoire électronique lisible et inscriptible (250).

8. Procédé de distribution de bandes de test (206) à partir du logement (212) d'une cartouche (19), comprenant les étapes suivantes:
faire coulisser un couvercle (202) depuis une première position, dans laquelle le couvercle ferme une ouverture (208) dans le logement et est engagé avec un joint (204) autour de l'ouverture, jusqu'à une seconde position, dans laquelle le couvercle est désengagé du joint et est engagé avec une bande de test (206) dans le logement;
dans lequel le couvercle présente une surface de coulissement (210) juxtaposée à une surface d'engagement (212) au niveau d'un bord de l'ouverture; et
dans lequel le procédé comprend en outre le coulissement du couvercle depuis la seconde position jusqu'à la première position de telle sorte que la bande de test soit forcée hors du logement par le couvercle; et
dans lequel la cartouche comprend en outre un moyen de poussée de couvercle pour pousser le couvercle contre le joint dans la première position,
**caractérisé en ce que**:
au moins une section (210a) de la surface de coulissement est inclinée par rapport à la surface d'engagement du bord d'ouverture de telle sorte que le coulissement du couvercle depuis la première position jusqu'à la seconde position désengage le couvercle du joint.
